# EUROPEAN PATENT SPECIFICATION

(11) **EP 1 356 299 B1**
(45) Date of publication and mention of the grant of the patent: **19.04.2006**
(21) Application number: 02705902.1
(22) Date of filing: 23.01.2002
(51) Int. Cl.: G01N 33/68

(54) **METHOD FOR THE DIAGNOSIS OF ALZHEIMER'S DISEASE**
VERFAHREN ZUR DIAGNOSE DER ALZHEIMERSCHEN KRANKHEIT
METHODE PERMETTANT DE DIAGNOSTIQUER LA MALADIE D'ALZHEIMER

(30) Priority: 23.01.2001 US 263841 P
(43) Date of publication of application: 29.10.2003
(73) Proprietor: MONASH UNIVERSITY, Clayton, Vic 3800 (AU)
(72) Inventor: SMALL, David, Henry, Ashburton, VIC 3147 (AU); FODERO, Lisa, Reservoir, VIC 3073 (AU)
(74) Representative: Woods, Geoffrey Corlett
(86) International application number: PCT/US2002/001874
(87) International publication number: WO 2002/059619

(56) References cited:
- WO-A-01/77370
- WO-A-99/15695
- SAEZ-VALERO J ET AL: "ALTERED GLYCOSYLATION OF CEREBROSPINAL FLUID BUTYRYLCHOLINESTERASE IN ALZHEIMER'S DISEASE" BRAIN RESEARCH, AMSTERDAM, NL, vol. 889, 19 January 2001 (2001-01-19), pages 247-250, XP002941860 ISSN: 0006-8993
- ARENDT T ET AL: "DECREASED RATIO OF CSF ACETYLCHOLINESTERASE TO BUTYRYLCHOLINESTERASE ACTIVITY IN ALZHEIMER'S DISEASE" LANCET THE, LANCET LIMITED. LONDON, GB, vol. 1, no. 8369, January 1984 (1984-01), page 173 XP002941861 ISSN: 0140-6736
- SAEZ-VALERO J ET AL: "MOLECULAR ISOFORM DISTRIBUTION AND GLYCOSYLATION OF ACETYLCHOLINESTERASE ARE ALTERED IN BRAIN AND CEREBROSPINAL FLUID OF PATIENTS WITH ALZHEIMER'S DISEASE" JOURNAL OF NEUROCHEMISTRY, NEW YORK, NY, US, vol. 72, 1999, pages 1600-1608, XP002941859 ISSN: 0022-3042
- SAEZ-VALERO JAVIER ET AL: "Glycosylation of acetylcholinesterase as diagnostic marker for Alzheimer's disease." LANCET (NORTH AMERICAN EDITION), vol. 350, no. 9082, 1997, page 929 XP000914161 ISSN: 0099-5355 cited in the application
- SAEZ-VALERO J ET AL: "Altered glycosylation of acetylcholinesterase in lumbar cerebrospinal fluid of patients with Alzheimer's disease." JOURNAL OF NEUROLOGY NEUROSURGERY & PSYCHIATRY, vol. 69, no. 5, November 2000 (2000-11), pages 664-667, XP001089627 ISSN: 0022-3050
- SZUMANSKA G ET AL: "LECTIN HISTOCHEMISTRY OF PLAQUES AND TANGLES IN ALZHEIMER'S DISEASE" ACTA NEUROPATHOLOGICA, vol. 73, no. 1, 1987, pages 1-11, XP001089624 ISSN: 0001-6322
- SPARKMAN D R ET AL: "PAIRED HELICAL FILAMENTS ARE NOT THE MAJOR BINDING SITES FOR WHEAT GERM AND DOLICHOS-BIFLORUS AGGLUTININS IN THE NEUROFIBRILLARY TANGLES OF ALZHEIMER'S DISEASE" ACTA NEUROPATHOLOGICA, vol. 79, no. 6, 1990, pages 640-646, XP001089623 ISSN: 0001-6322
- SAEZ-VALERO JAVIER ET AL: "Acetylcholinesterase and butyrylcholinesterase glycoforms are biomarkers of Alzheimer's disease." JOURNAL OF ALZHEIMER'S DISEASE, vol. 3, no. 3, June 2001 (2001-06), pages 323-328, XP001089626 ISSN: 1387-2877

## Description

### FIELD OF THE INVENTION

This invention relates to a method for diagnosing Alzheimer's Disease in patients using multiple biomarkers. More specifically, the present invention provides a method for monitoring the levels of glycoproteins in patients suffering from such disorders by using wheat germ agglutinin as a biomarker to specifically bind to glycoproteins isolated from cerebrospinal fluid (CSF). In addition, this method combines the measurements of wheat germ agglutinin-reactive glycoproteins in CSF with alteration in glycosylation patterns in such patients.

### BACKGROUND OF THE INVENTION

Presently, the only diagnosis of a number of prion related disorders including transmissible spongiform encephalopathies (TSE) and dementias found, e.g., in Alzheimer's Disease (AD) is neuropsychological assessment by *post mortem* examination of the brain. A common problem in interpreting *ante mortem* cerebrospinal fluid (CSF) results has been the uncertainty of clinical diagnosis. It has been estimated that the accuracy of diagnosis of AD by clinical examination is only about 80-90% at best (Growdonk 1999). Therefore, some individuals diagnosed as AD may be suffering from a non-AD dementia and some of the non-AD samples may be from individuals with latent AD. Therefore, there is a need to identify specific biochemical markers of AD and other dementias and prion related disorders.

Studies have shown that the.level of tau (Ara et al., 1995; Tojanowski et al., 1996) and AB (Nakamura et al., 1994; Motter et al., 1995; Galasko et al., 1998; Kani et al., 1998) are altered in AD CSF. Several studies have demonstrated that the use of multiple biomarkers in combination may improve diagnosis. (Galasko et al., 1998; Kanai et al., 1998). However, the present biomarkers do not provide the sensitivity or specificity for AD diagnosis (Working Group et al., 1998).

The glycosylation patterns of many proteins are altered in severe disease states (Lis & Sharon, 1993) including Alzheimer's Disease (AD). The lectin blotting technique has been used to examine the glycosylation of proteins in non-AD and AD CSF (Savage et al., 1992). Alterations in the glycosylation pattern of several proteins in the AD brain have been studied (Guevara et al., 1998; McFarlane et al., 1999). Previous studies demonstrated that the level of an unusual glycoform of acetylcholinesterase (AChE) is increased in the AD brain and CSF (Saez-Valero et al., 1993, 1997). This glycoform can be distinguished from other AChE isoforms because this unusual isoform does not bind to the lectin, *Conavalia ensiformis* (ConA). However, until the present invention, changes in AChE expression and glycoform distribution have not been found to be of sufficient sensitivity or specificity to be useful diagnostic markers for AD.

Alteed glycosylation of CSF butyrylcholinesterase has been studied in Alzheimer's Disease (Saez-Valero et al., 2001), Lectin histochemistry has also been employed in the study of Alzheimer's Disease (Szumanska et al., 1987).

### SUMMARY OF INVENTION

The present invention relates to a method for diagnosing Alzheimer's Disease, by measuring the level of glycoproteins isolated from fluid from subjects with the above conditions that bind to wheat germ agglutinin (WGA).

In addition, the present invention provides a method for increasing the sensitivity for diagnosing Alzheimer's Disease by combining the use of measuring the levels of glycoproteins bound to wheat germ agglutinin with the assay for measuring the binding of AChE isoform with ConA.

The present invention further provides a method for increasing the sensitivity for diagnosing Alzheimer's Disease by combining the use of measuring the levels of glycoproteins bound to wheat germ agglutinin with the assay for measuring the binding of butyrylcholinesterase with ConA.

### DETAILED DESCRIPTION OF THE FIGURES

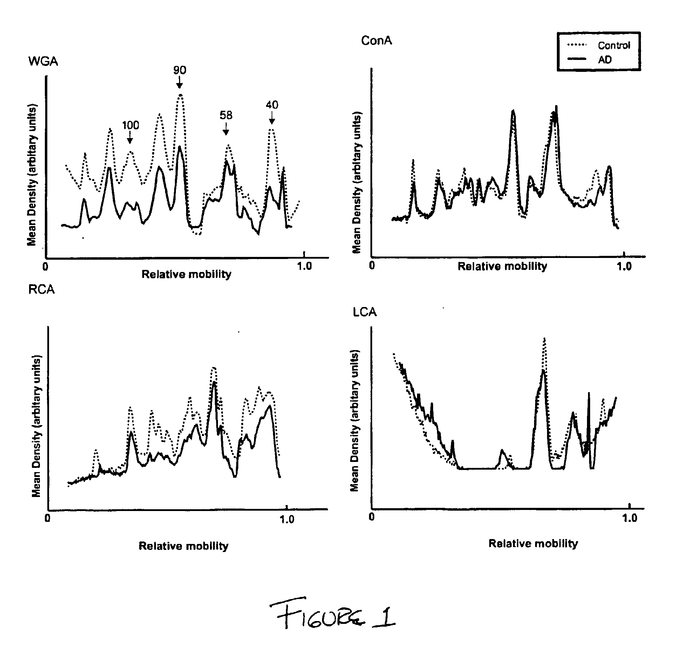

**Figure 1** shows the densitometric profile of the staining pattern after analysis of a typical AD CSF sample and a non-AD CSF sample by SDS-PAGE. Arrows indicate major WGA-reactive glycoproteins, with apparent molecular weights of 58 K, 90 K and 100 K, which are decreased in the AD samples.

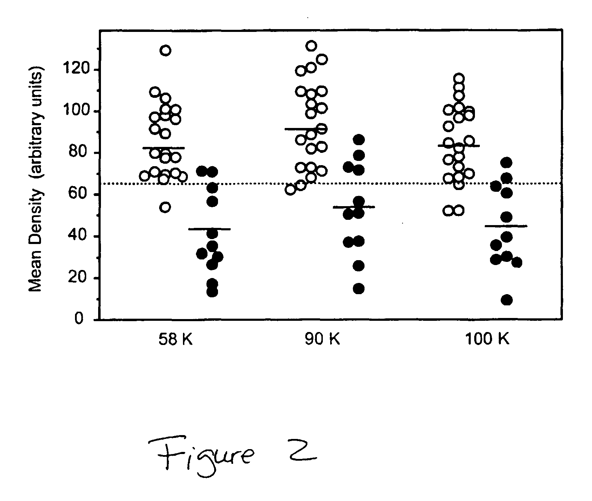

**Figure 2** shows quantitative analysis of three WGA -reactive glycoproteins (58 K, 90 K and 100 K) in non-AD (o) and AD (•) CSF samples. The intensity of all three biotinylated protein bands was significantly lower in AD CSF than in controls (P< 0. 001, Student's *t* test). Mean density values ± SEM for the three WGA-reactive glycoproteins were non-AD: 58 K 82 ±4, 90 K 91 ±4, 100 K 82 ±3, AD: 58 K 42 ±6, 90 K 53 ±7, 100 K 44 ±6. Mean value was calculated (WGA staining density of 58 K band +WGA staining density of 90 K band + WGA staining density of 100 K band / 3). The value of W in AD CSF was approximately 50 % lower than controls.

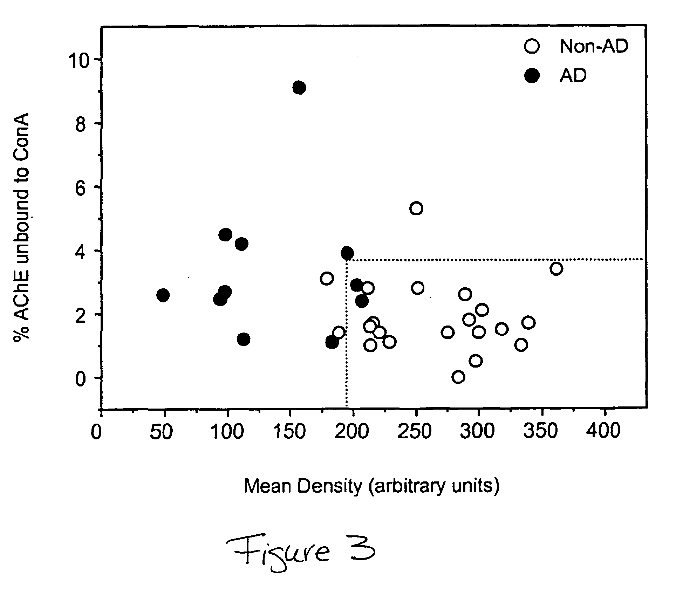

**Figure 3** shows a plot of the sum of the mean densities for WGA staining of three WGA -reactive glycoproteins (W) versus % AchE unbound to *Conavalia ensiformis* (ConA) in AD(•) and non- AD *(0) ante mortem* CSF samples. Dotted lines show cut-off values which discriminates between probable AD and non-AD samples.

### DETAILED DESCRIPTION OF THE INVENTION

The present invention provides a method for the diagnosis of Alzheimer's Disease in a patient comprising the steps of providing a sample of an appropriate body fluid from the patient, isolating proteins from the sample, labeling WGA with biotin or any convenient label, mixing the sample with WGA, determining the level of WGA binding and comparing the level of WGA binding to a known standard.

In a preferred embodiment, the relative amount of proteins that bind to WGA is measured. Measurement of the reactive bound WGA-protein may be carried out in any convenient manner for example, by using biochemical analysis such as HPLC, mass spectrometry or immunological techniques such as ELISA, or assays. However, a particularly preferred means of measuring the WGA-reactive proteins involves a lectin-binding analysis.

The body fluid analyzed can be cerebrospinal fluid (CSF), blood or blood plasma.

The method of the present invention is used for diagnosis of Alzheimer's Disease. The present inventors demonstrated that the levels of glycosylation patterns which bound to the WGA were decreased in patients with AD CSF compared to non-AD controls. More importantly, the glycosylation patterns of CSF proteins that bound with other lectins did not differ between non-AD and AD samples. Therefore the present invention is used for the diagnosis of Alzheimer's Disease.

The present invention further provides a method for increasing the sensitivity and specificity for diagnosis of Alzheimers Disease by using multiple biomarkers. In particular, in another embodiment, the level of WGA binding to glycoproteins isolated from samples from subjects manifesting conditions of dementia or prion disease is compared to the percentage of acetylcholinesterase (AChE) unbound to ConA.

Previous AD studies have revealed an increase in an unusual glycosylated isoform of a AChE in AD CSF that does not bind ConA. Therefore, in a preferred embodiment, the method of comparing the level of WGA binding to glycoproteins isolated from samples from subjects manifesting conditions of dementia to the percentage of AChE unbound to ConA to increase sensitivity and specificity is used to diagnose Alzheimer's Disease.

In still another embodiment, the present invention provides a method for increasing the sensitivity and specificity for diagnosis of Alzheimer's Disease by comparing the level of WGA binding to glycoproteins isolated from samples from subjects manifesting conditions of dementia to the percentage of butyrylcholinesterase (BuChE) unbound to ConA.

It has been established that approximately 93.6% of the BuChE in CSF of AD subjects binds ConA. Accordingly, in a preferred embodiment, the method of comparing the level of WGA binding to glycoproteins isolated from samples from subjects manifesting conditions of dementia or prion disease to the percentage of BuChE unbound to ConA to increase sensitivity and specificity is used for the diagnosis of Alzheimer's Disease.

### EXAMPLES

### Materials and Methods

### I. Collection of CSF samples

CSF samples from 21 non -AD (5 non -neurological diseases, 5 epilepsy, 5 hydrocephalus, 3 polyneuropathies and 3 intracranial hypertension: mean age 71± 3 years) and 11 probable AD (mean age 62 ± 6 years) cases were collected from patients at the Hospital Universitario San Carlos of Madrid. Patients were diagnosed for probable AD according to NINCDS-ADRDA criteria (McKhann et al., 1984). The duration of disease from the Alzheimer's Disease group was [mean (SEM)] 1.9 (0.3) years and the average clinical dementia rating (CDR) was 1.4 (0.18). Lumbar punctures were performed in lateral decubitus and the CSF was stored immediately at -40°C. No drugs were taken 12 hours before the collection of CSF. None of the patients received anti-cholinesterase therapy.

There was no significant difference in total protein concentration in non-AD CSF or AD CSF (non-AD CSF 1.05 mg/mL ± 0.08, AD CSF 0.96 mg/mL ± 0.12, P = 0.546) as determined by the bicinchoninic acid (BCA) method(Smith et al., 1985).

### II. Lectin blotting

CSF was mixed with an equal volume of a sodium dodecyl sulfate-polyacrylamide gel electrophoresis (SDS-PAGE) sample buffer containing 1 M Tris-HCl (pH 6.8), 4%(w/v)SDS, 2%(w/v) glycine,0.01% (w/v) bromophenol blue and 10%(v/v) β-mercaptoethanol and heated in a boiling water bath for five minutes. Aliquots (15 µL) of this mixture were applied to 7.5% SDS-PAGE. After electrophoresis proteins were transferred onto polyvinyllidene difluoride (PVDF) membranes at a constant current of 300 mA overnight. Membranes were blocked in 0.5% hydrolyzed casein in phosphate-buffered saline (PBS), pH 7.4,for 1 h. The blocked membranes were then incubated with 12.5 µg/mL biotin-labeled lectins, *Triticum vulgaris* agglutinin (wheat germ; WGA), *Conavalia ensiformis* agglutinin (ConA), *Ricinus communis* agglutinin (RCA₁₂₀) or *Lens culinaris* agglutinin (LCA) (Sigma-Aldrich Pty. Ltd., Seven Hills, Australia) in Tris-buffered saline-Tween20 (polyoxyethelyene-sorbiton monolaurate) (T BST) for 1 h at room temperature. After washing the membranes with TBST, glycoproteins were visualized using (1:1000 dilution) NeutrAvidin-conjugated to alkaline phosphatase (Pierce Chemicals Co., Rockford, Illinois, USA) and developed with a mixture of Fast Red (6 mg/mli n AP buffer) and naphthol AS-MX substrate (0.4 mg/ml in distilled water). The stained blots were scanned using a UMAX Astra 2400S scanner and bands from lectin blots were quantified by densitometry using the NIH Image (Version 1.57) program written by W. Rasband (National Institutes of Health, Bethesda, MD). Peak areas were defined as the area under the curve between two minima after subtracting a background value obtained from a blank region of the blot.

### Example I Wheat Germ Agglutinin-Reactive glycoproteins (WGA binding index)

The staining of CSF proteins with biotinylated WGA was found to be less intense in the AD samples than in non-AD controls. However, the staining of CSF proteins with other lectins (ConA, LCA or RCA) did not differ between non-AD and AD samples (see, Fig. 1).

Several WGA-reactive proteins were decreased in AD CSF. Three of the most intensively stained glycoproteins, with apparent molecular weights of 58 K, 90 K and 100 K, were chosen for quantitative analysis (see, Fig. 2). The staining of all three glycoproteins with WGA was significantly decreased (P<0.001 as assessed by the Student's t test) in AD CSF compared to controls (see, Fig. 2). The percentage decrease in AD CSF was approximately 30 %, 40 % and 45 % for the 58 K, 90 K and 100 K proteins, respectively. As all three WGA glycoproteins were decreased in AD CSF, a mean value (W) for the density of all three bands was calculated (WGA staining density of 58 K band + WGA staining density of 90 K band + WGA staining density of 100 K band / 3)(see, Fig. 2). The value of W in AD CSF was approximately 50 % lower than controls.

### Example II Use of Multiple AD Biomarkers

To increase the sensitivity and specificity of the present method for diagnosis of AD, the use of multiple biomarkers was explored by combining the measurements of AChE glycosylation with the WGA binding index. The measurements of the percentage of AChE unbound to ConA (an index of the unusual AChE isoform) were plotted against the staining intensity of the sum of the mean densities (mean density values of 58 K + 90 K + 100 K) of the three proteins detected by biotinylated WGA (see, Fig. 3). The majority of non-AD samples were situated in the bottom right hand corner of the plot. The sensitivity and specificity of the combined measurements were calculated by drawing a dotted line separating the non-AD group and AD group (see Fig. 3). The sensitivity of the test was defined by the number of non-AD measurements in the AD boundary and the specificity was defined as the number of AD measurements in the non- AD boundary. The combined measurements of the alterations in the glycoproteins (WGA-reactive index and AChE isoform index) was 82% and 86% detection for sensitivity and specificity, respectively.

### References

1. Arai, H. , Terajima, M. , Miura, M. , et al. (1995) Tau in cerebrospinal fluid: a potential diagnostic marker in Alzheimer's Disease. Ann *Neurol* **38**(4): 649-652.
2. Davies, P. , Gilman, S. , Growdon, J. H. , et al. (1998) Consensus report of the Working Group on: "Molecular and Biochemical Markers of Alzheimer's Disease". The Ronald and Nancy Reagan Research Institute of the Alzheimer's Association and the National Institute on Aging Working Group. *Neurobiol Aging* **19**(2): 109-116.
3. Elgavish, S, and Shaanan, B. (1997) Lectin-carbohydrate interactions: different folds, common recognition principles. *TIBS* **22**(12): 462-467.
4. Ellman, G. L. , Courtney, K. D. , Andros. V. Jr. , Featherstone, R. M. (1961) A new and rapid colorimetric determination of acetylcholinesterase activity. *Biochem Pharmacol* **7**: 88-95.
5. Galasko, D. , Chang, L. , Motter, R. , et al., (1998) High cerebrospinal fluid *tau* and low amyloid P42 levels in the clinical diagnosis of Alzheimer's Disease and relation to apolipoprotein E genotype. *Arch Neurol* **55**(7): 937-945.
6. Growdon, J. H. (1999) Biomarkers of Alzheimer's Disease. Arch. Neurol **56**(3): 281-283.
7. Kanai, M. , Matsubara, E. , Isoe, K. , et al., (1998) Longitudinal study of cerebrospinal fluid levels of tau, AP 1-40, and Ap 142(43) in Alzheimer's Disease: a study in Japan. *Ann Neurol* **44**(1): 17-26.
8. Lis, H. , Sharon, N. Lectins as molecules and as tools. (1986) *Annu Rev Biochem* **55:** 35-67.
9. Lis, H. , Sharon, N. (1993) Protein glycosylation. Structural and functional aspects. *Eur J Biochem* **218**(1): 1-27.
10. McKhann, G. , Drachman, D. , Folstein, M. , et al. (1984) Clinical diagnosis of Alzheimer's Disease: report of the NINCDS-ADRDA Work Group under the auspices of Department of Health and Human Services Task Force on Alzheimer's Disease. *Neurology* **34**(7): 939-944.
11. Monsigny, M. , Roche, A. C. , Sene, C. , et al. (1980) Sugar-lectin interactions: how does wheat-germ agglutinin bind sialoglycoconjugates? *Eur J Biochem* **104**(1): 147-153.
12. Motter, R. , Vigo-Pelfrey, C. , Kholodenko, D. , et al. (1995) Reduction of P-amyloid peptide42 in the cerebrospinal fluid of patients with Alzheimer's Disease. *Ann Neurol* **38**(4): 643-648.
13. Nagata, Y. , and Burger, M. M. Wheat germ agglutinin. (1974) Molecular characteristics and specificity for sugar binding. *J Biol Chem* **249**(10): 3116-3122.
14. Nakamura, T. , Shoji, M., Harigaya, Y. , et al. (1994) Amyloid P protein levels in cerebrospinal fluid are elevated in early-onset Alzheimer's Disease. Ann *Neurol* **36**(6): 903-911.
15. Saez-Valero, J. , Tomel, P. L. , Munoz-Delgado, E. et al. (1993) Amphiphilic and hydrophilic acetyl- and butyrylcholinesterase in human brain. *J Neurosci Res* **35**(6): 678-689.
16. Saez-Valero, J. , Sberna, G. , McLean, C. A. , et al. (1997) Glycosylation of acetylcholinesteraseas a diagnostic marker for Alzheimer's Disease. *Lancet* **350**(9082): 929.
17. Saez-Valeo, J. et al. (2001) Alteed glycosylation of cerebrospinal fluid butyrylcholinesterase in Alzheimer's Disease, Brain Research 889(19) pp 247-250
18. Savage, D. , Mattson, G. , Desai, S. , et al. (1992) Avidin-Biotin Chemistry: AH and book, Pierce Chemical Company, Rockford, Illinois pp. 138-139.
19. Szumanska G., Vorbrodt A.W., Mandybar T.I. and Wisniewski (1987) Lectin histochemistry of plaques and bangles in Alzheimer's Disease, Acta Neuropathologica 73(1) pp I-II.

## Claims

1. A method for diagnosing Alzheimer's Disease comprising the steps of:
(a) providing a sample of an appropriate body fluid;
(b) isolating protein from the sample;
(c) mixing the protein with labeled wheat germ agglutinin;
(d) detecting the level of binding of the proteins with the labeled wheat germ agglutinin; and
(e) comparing result of step (d) with the level of a known standard.

2. The method according to claim 1, wherein the body fluid is cerebrospinal fluid, blood or blood plasma.

3. The method according to claim 1, wherein the wheat germ agglutinin is labeled with biotin.

4. A method for diagnosing Alzheimer's Disease comprising the steps of:
(a) providing a first sample of an appropriate body fluid;
(b) isolating protein from the first sample;
(c) mixing the protein with labeled wheat germ agglutinin;
(d) detecting the level of binding of the proteins with the labeled wheat germ agglutinin;
(e) comparing step (d) with the level of a known standard;
(f) combining a second sample of the appropriate body fluid with ConA;
(g) measuring the percentage of acetylcholinesterase bound to ConA of the second sample;
(h) measuring the percentage of acetylcholinesterase unbound to ConA of the second sample; and
(i) calculating the ration of the acetylcholinesterase unbound to the ConA of the second sample to the level of binding of the proteins with the labeled wheat germ agglutinin of the first sample.

5. A method according to claim 4 wherein the body fluid is cerebrospinal fluid, blood or blood plasma.

6. A method according to claim 4 wherein the wheat germ agglutinin is labeled with biotin.

7. A method for diagnosing Alzheimer's Disease comprising the steps of:
(a) providing a first sample of an appropriate body fluid;
(b) isolating protein from the first sample;
(c) mixing the protein with labeled wheat germ agglutinin;
(d) detecting the level of binding of the proteins with the labeled wheat germ;
(e) comparing step (d) with the level of a known standard;
(f) combining a second sample of the appropriate body fluid with ConA;
(g) detecting the presence if butyrylcholinesterase with an altered glycosylation pattern binding to ConA of the second sample;
(h) measuring the percentage of butyrylcholinesterase unbound to ConA of the second sample; and
(i) calculating the ratio of the butyrylcholinesterase unbound to the ConA of the second sample to the level of binding of the proteins with the labeled wheat germ agglutinin of the first sample.

8. A method according to claim 7 wherein the body fluid is cerebrospinal fluid, blood or blood plasma.

9. A method according to claim 7 wherein the wheat germ agglutinin is labeled with biotin.

10. The method according to any of claim 1, claim 4 or claim 7, wherein the samples are isolated from Alzheimer's Disease subjects.

## Revendications

1. Procédé de diagnostic de la maladie d'Alzheimer, comprenant les étapes consistant à :
(a) obtenir un échantillon d'un fluide corporel approprié ;
(b) isoler une protéine de l'échantillon ;
(c) mélanger la protéine avec de l'agglutinine de germe de blé marquée ;
(d) détecter le taux de liaison des protéines à l'agglutinine de germe de blé marquée ; et
(e) comparer le résultat de l'étape (d) avec le taux d'un étaltin connu.

2. Procédé selon la revendication 1, dans lequel le fluide corporel est du liquide céphalorachidien, du sang ou du plasma sanguin.

3. Procédé selon la revendication 1, dans lequel l'agglutinine de germe de blé est marquée à la biotine.

4. Procédé de diagnostic de la maladie d'Alzheimer, comprenant les étapes consistant à :
(a) obtenir un premier échantillon d'un fluide corporel approprié ;
(b) isoler une protéine du premier échantillon ;
(c) mélanger la protéine avec de l'agglutinine de germe de blé marquée ;
(d) détecter le taux de liaison des protéines à l'agglutinine de germe de blé marquée ;
(e) comparer l'étape (d) avec le taux d'un étalon connu ;
(f) combiner un deuxième échantillon du fluide corporel approprié avec de la ConA ;
(g) mesurer le pourcentage d'acétylcholinestérase liée à la ConA du deuxième échantillon ;
(h) mesurer le pourcentage d'acétylcholinestérase non liée à la ConA du deuxième échantillon ; et
(i) calculer le ratio de l'acétylcholinestérase non liée à la ConA du deuxième échantillon sur le taux de liaison des protéines à l'agglutinine de germe de blé marquée du premier échantillon.

5. Procédé selon la revendication 4, dans lequel le fluide corporel est du liquide céphalorachidien, du sang ou du plasma sanguin.

6. Procédé selon la revendication 4, dans lequel l'agglutinine de germe de blé est marquée à la biotine.

7. Procédé de diagnostic de la maladie d'Alzheimer, comprenant les étapes consistant à :
(a) obtenir un premier échantillon d'un fluide corporel approprié ;
(b) isoler une protéine du premier échantillon ;
(c) mélanger la protéine avec de l'agglutinine de germe de blé marquée ;
(d) détecter le taux de liaison des protéines à l'agglutinine de germe de blé marqué ;
(e) comparer l'étape (d) avec le taux d'un étalon connu ;
(f) combiner un deuxième échantillon du fluide corporel approprié avec de la ConA ;
(g) détecter la présence de butyrylcholinestérase par une liaison à la ConA selon un schéma de glycosylation modifié du deuxième échantillon ;
(h) mesurer le pourcentage de butyrylcholinestérase non liée à la ConA du deuxième échantillon ; et
(i) calculer le ratio de la butyrylcholinestérase non liée à la ConA du deuxième échantillon sur le taux de liaison des protéines à l'agglutinine de germe de blé marquée du premier échantillon.

8. Procédé selon la revendication 7, dans lequel le fluide corporel est du liquide céphalorachidien, du sang ou du plasma sanguin.

9. Procédé selon la revendication 7, dans lequel l'agglutinine de germe de blé est marquée à la biotine.

10. Procédé selon l'une quelconque de la revendications 1, de la revendication 4 ou de la revendication 7, dans lequel les échantillons sont isolés de sujets atteints de la maladie d'Alzheimer.

## Patentansprüche

1. Verfahren zur Diagnose der Alzheimerschen Krankheit, umfassend die folgenden Schritte:
(a) Bereitstellen einer Probe einer geeigneten Körperflüssigkeit;
(b) Isolieren von Protein aus der Probe;
(c) Mischen des Proteins mit markiertem Weizenkeimagglutinin;
(d) Detektieren des Ausmaßes der Bindung der Proteine mit dem markierten Weizenkeimagglutinin; und
(e) Vergleichen des Ergebnisses von Schritt (d) mit dem Wert eines bekannten Standards.

2. Verfahren gemäß Anspruch 1, wobei die Körperflüssigkeit cerebrospinale Flüssigkeit, Blut oder Blutplasma ist.

3. Verfahren gemäß Anspruch 1, wobei das Weizenkeimagglutinin mit Biotin markiert ist.

4. Verfahren zum Diagnostizieren der Alzheimerschen Krankheit, umfassend die folgenden Schritte:
(a) Bereitstellen einer ersten Probe einer geeigneten Körperflüssigkeit;
(b) Isolieren von Protein aus der ersten Probe;
(c) Mischen des Proteins mit markiertem Weizenkeimagglutinin;
(d) Detektieren des Ausmaßes der Bindung der Proteine mit dem markierten Weizenkeimagglutinin;
(e) Vergleichen von Schritt (d) mit dem Wert eines bekannten Standards;
(f) Zusammenbringen einer zweiten Probe der geeigneten Körperflüssigkeit mit ConA;
(g) Messen des Prozentanteils an Acetylcholinesterase, die an ConA der zweiten Probe gebunden ist;
(h) Messen des Prozentanteils an Acetylcholinesterase, die nicht an ConA der zweiten Probe gebunden ist; und
(i) Berechnen des Verhältnisses der nicht an dem ConA der zweiten Probe gebundenen Acetylcholinesterase zu dem Ausmaß der Bindung der Proteine mit dem markierten Weizenkeimagglutinin der ersten Probe.

5. Verfahren gemäß Anspruch 4, wobei die Körperflüssigkeit cerebrospinale Flüssigkeit, Blut oder Blutplasma ist.

6. Verfahren gemäß Anspruch 4, wobei das Weizenkeimagglutinin mit Biotin markiert ist.

7. Verfahren zum Diagnostizieren der Alzheimerschen Krankheit, umfassend die folgenden Schritte:
(a) Bereitstellen einer ersten Probe einer geeigneten Körperflüssigkeit;
(b) Isolieren von Protein aus der ersten Probe;
(c) Mischen des Proteins mit markiertem Weizenkeimagglutinin;
(d) Detektieren des Ausmaßes der Bindung der Proteine mit dem markierten Weizenkeimagglutinin;
(e) Vergleichen von Schritt (d) mit dem Wert eines bekannten Standards;
(f) Zusammenbringen einer zweiten Probe der geeigneten Körperflüssigkeit mit ConA;
(g) Detektieren der Anwesenheit von Butyrylcholinesterase mit einem veränderten Glykosylierungsmuster, die an ConA der zweiten Probe bindet;
(h) Messen des Prozentanteils an Butyrylcholinesterase, die nicht an ConA der zweiten Probe gebunden ist; und
(i) Berechnen des Verhältnisses der nicht an dem ConA der zweiten Probe gebundenen Butyrylcholinesterase zu dem Ausmaß der Bindung der Proteine mit dem markierten Weizenkeimagglutinin der ersten Probe.

8. Verfahren gemäß Anspruch 7, wobei die Körperflüssigkeit cerebrospinale Flüssigkeit, Blut oder Blutplasma ist.

9. Verfahren gemäß Anspruch 7, wobei das Weizenkeimagglutinin mit Biotin markiert ist.

10. Verfahren gemäß Anspruch 1, Anspruch 4 oder Anspruch 7, wobei die Proben aus an der Alzheimerschen Krankheit erkrankten Patienten isoliert werden.
